# EUROPEAN PATENT APPLICATION

(11) **EP 4 588 821 A1**
(43) Date of publication of application: **23.07.2025**
(21) Application number: 23864960.2
(22) Date of filing: 07.04.2023
(51) Int. Cl.: B65D 75/62, B65D 85/07

(54) **ABSORBENT ARTICLE PACKAGING POUCH AND ABSORBENT ARTICLE PACKAGING PRODUCT**

(30) Priority: 15.09.2022 JP 2022146677
(71) Applicant: DAIO PAPER CORPORATION, Shikokuchuo-shi Ehime 799-0492 (JP)
(72) Inventor: TAKAHIRA, Akira, Sakura-shi, Tochigi 329-1411 (JP)
(74) Representative: Epping - Hermann - Fischer
(86) International application number: PCT/JP2023/014330
(87) International publication number: WO 2024/057594

(57) **Abstract**

An absorbent article packaging bag contains multiple absorbent article arrays in an up-down direction, each array being formed with multiple absorbent articles standing erect and aligned, and:
the absorbent article packaging bag is substantially a cuboid in shape, with a top face part, a bottom face part, and enclosing face parts connecting the top face part and the bottom face part;
tearable parts, from which the enclosing face parts can be torn in the up-down direction, are formed in the enclosing face parts;
the tearable parts include a first tearable part, formed within an extent of one absorbent article array in the up-down direction, and a second tearable part, formed apart from the first tearable part in the up-down direction and formed within an extent of another absorbent article array in the up-down direction; and
the first tearable part has lower breaking strength than the second tearable part.

## Description

### TECHNICAL FIELD

The present invention relates to an absorbent article packaging bag and an absorbent article package.

### BACKGROUND ART

Generally, absorbent articles such as incontinence pads, sanitary napkins, and disposable diapers are folded, wrapped individually on an as-needed basis, and provided as individually-wrapped articles. A number of such absorbent articles or individually-wrapped articles are put in a packaging bag and provided thus.

The packaging bag is provided with a seam-like part (tearable part), which acts like a perforation and from which the packaging bag can be torn open with ease. Many of these packaging bags are structured such that an opening, from which the individually-wrapped articles contained inside can be taken out, can be formed by tearing open the packaging bag at the seam-like part. Also, structures in which a number of tearable parts like this are formed in a packaging bag are also known (see, for example, patent document 1).

### RELATED-ART DOCUMENT

### PATENT DOCUMENT

[Patent Document 1] Unexamined Japanese Patent Application Publication No. 2003-292043

### SUMMARY OF THE INVENTION

### PROBLEM TO BE SOLVED BY THE INVENTION

As mentioned above, although structures with multiple tearable parts have been known heretofore, the relationship between the tearable parts, such as how breaking strength varies between them, has not been studied sufficiently yet. When opening an absorbent article package for the first time, the user usually tears open the packaging bag at one tearable part, and is careful not to tear other tearable parts. However, depending on the force the user uses to tear open the packaging bag, the way the user tears open the packaging bag, and so forth, even if the user intends to tear only one tearable part, he/she might unintentionally tear other tearable parts as well. Therefore, the user has to be careful not to tear open the packaging bag in an unintended way. Therefore, there is a need for a structure that makes it easy to tear open the packaging bag when starting to use the absorbent article package.

In view of the foregoing, embodiments of the present invention aim to provide an absorbent article packaging bag having multiple tearable parts and structured such that the packaging bag can be torn open with ease at the beginning of use.

### MEANS TO SOLVE THE PROBLEM

An embodiment of the present invention provides an absorbent article packaging bag configured to contain two or more absorbent article arrays in an up-down direction, each one of the two or more absorbent article arrays being formed with a plurality of absorbent articles that stand erect and are aligned, and, in this absorbent article packaging bag:
the absorbent article packaging bag is substantially a cuboid in shape, with a top face part, a bottom face part, and enclosing face parts connecting the top face part and the bottom face part,
tearable parts, from which the enclosing face parts can be torn in the up-down direction, are formed in the enclosing face parts,
the tearable parts include:
   a first tearable part, formed within an extent of one of the two or more absorbent article arrays in the up-down direction; and
   a second tearable part, formed apart from the first tearable part in the up-down direction, and formed within an extent of an absorbent article array other than the one of the two or more absorbent article arrays in the up-down direction, and
the first tearable part has lower breaking strength than the second tearable part.

### EFFECTS OF THE INVENTION

According to one embodiment of the present invention, it is possible to provide an absorbent article packaging bag having multiple tearable parts and structured such that the packaging bag can be torn open with ease at the beginning of use.

### BRIEF DESCRIPTION OF THE DRAWINGS

[FIG. 1] FIG. 1 is a perspective view of an absorbent article package according to a first embodiment of the present invention;
[FIG. 2] FIG. 2 is an exploded view of the absorbent article packaging bag of FIG. 1;
[FIG. 3] FIG. 3 is a diagram that explains the usage of the absorbent article package of FIG. 1;
[FIG. 4] FIG. 4 is a diagram that explains the usage of the absorbent article package of FIG. 1;
[FIG. 5] FIG. 5 is a perspective view of an absorbent article package according to a second embodiment of the present invention;
[FIG. 6] FIG. 6 is a diagram that illustrates an absorbent article package according to a variation;
[FIG. 7] FIG. 7 is a diagram that illustrates an absorbent article package according to a variation;
[FIG. 8] FIG. 8 is a diagram that illustrates an absorbent article package according to a variation; and
[FIG. 9] FIG. 9 is a diagram corresponding to a partially enlarged view of part I in FIG. 1.

### MODE FOR CARRYING OUT THE INVENTION

Embodiments of the present invention will be described below with reference to the accompanying drawings. Note that, unless otherwise specified, parts that are the same or substantially the same will be assigned the same reference codes in each drawing, without repeating their explanation.

### <First embodiment>

### (Absorbent article package)

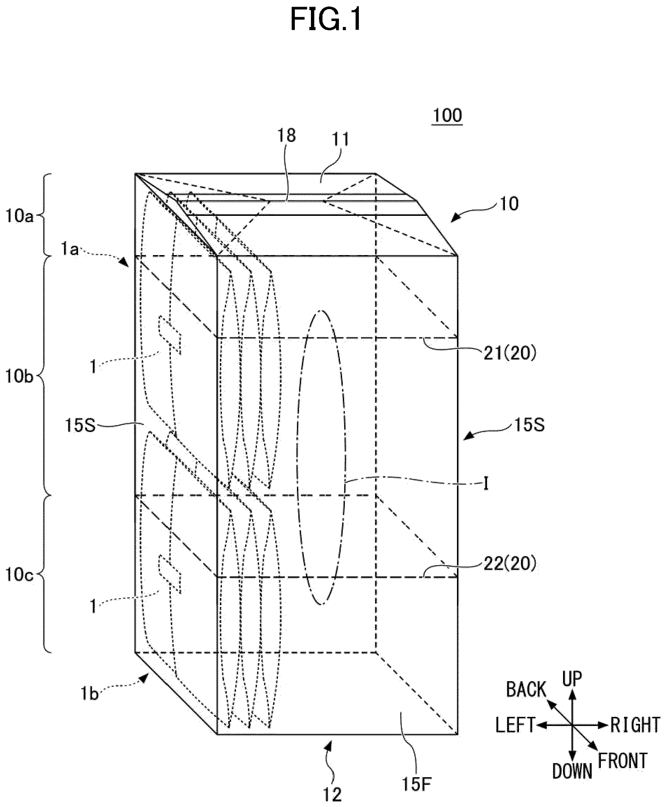

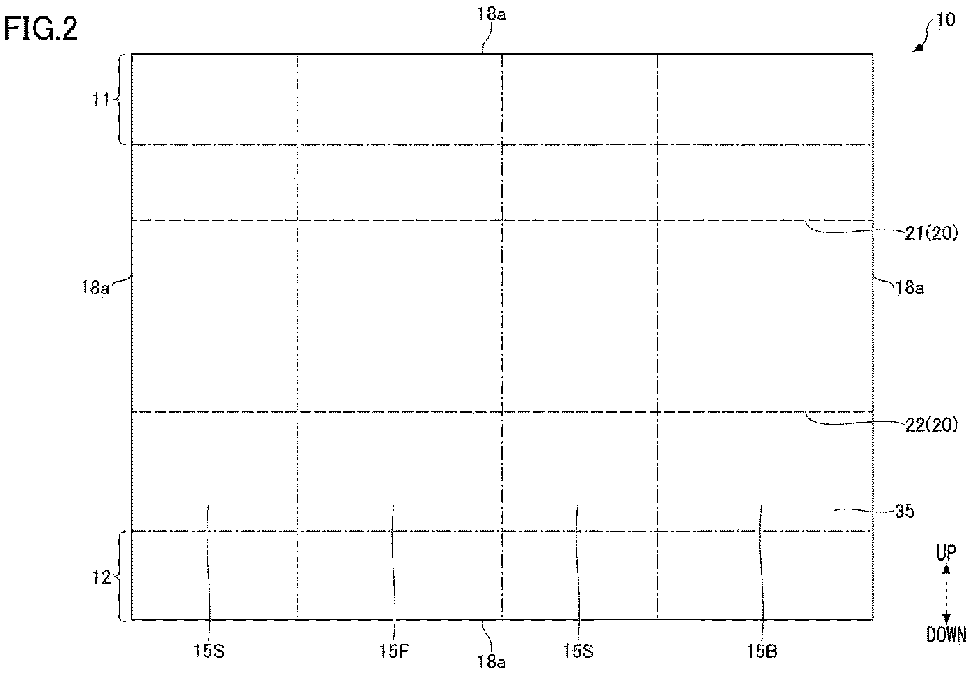

FIG. 1 shows an absorbent article package 100 according to an embodiment of the present invention, which includes an absorbent article packaging bag 10 (hereinafter also simply referred to as "packaging bag"), and multiple absorbent articles 1 contained in the absorbent article packaging bag 10. Also, FIG. 2 shows an expanded view of the absorbent article packaging bag 10 of FIG. 1. The absorbent article 1 may be a sanitary napkin, a panty liner, an incontinent pad, a disposable diaper, etc. The absorbent article 1 may be structured to include a liquid-permeable top sheet, a liquid-impermeable back sheet, and an absorber positioned between these sheets. In the illustrated example, the absorbent article 1 is an incontinence pad.

As shown in FIG. 1, multiple absorbent articles 1 are contained in the absorbent article packaging bag 10 such that the absorbent articles 1, standing erect, are aligned in the thickness direction of the absorbent articles 1. In this specification, the direction in which the absorbent articles 1 stand erect is referred to as "up-down direction," the direction in which the absorbent articles 1 are lined up is referred to as "left-right direction (horizontal direction)," and the direction orthogonal to the up-down direction and the left-right direction is referred to as "front-back direction." Also, usually, information about the absorbent articles (product name, logo, product type, etc.) is affixed to the absorbent article packaging bag. The direction which the user looking at this information in an ordinary way perceives as the up-down direction is the up-down direction of the absorbent article packaging bag 10 or the absorbent article package 100. In the present embodiment, absorbent articles 1 are contained in two levels, the upper level and the lower level. In the example shown in FIG. 1, the packaging bag 10 contains an upper array 1a of absorbent articles 1 (also referred to as "absorbent article array") and a lower array 1b of absorbent articles 1. However, the number of absorbent article arrays is by no means limited to two, and three or more upper and lower absorbent article arrays may be positioned over each other.

Referring to the example of FIG. 1, every absorbent article 1 is folded and wrapped individually with a wrapping sheet (therefore also referred to as "individually-wrapped article"). The individually-wrapped article of absorbent articles 1 may have dimensions of 50 mm to 200 mm in the longitudinal direction, 50 mm to 200 mm in the horizontal direction, and 3 mm to 30 mm in the thickness direction. Also, the absorbent articles 1 do not necessarily have to be wrapped individually. That is, an absorbent article 1 in this specification may refer to either an absorbent article that is not wrapped individually, or an absorbent article that is wrapped individually (individually-wrapped article with an absorbent article inside).

### (Packaging bag)

As shown in FIG. 1, the packaging bag 10 is substantially a cuboid in shape as a whole. In this specification, "substantially a cuboid in shape" means that the packaging bag 10 is a polyhedron having six rectangular faces (or square faces). The substantially cuboid shape does not necessarily have to be one that has distinct edges and/or vertices, and the edges and/or vertices may be rounded. That is, any three-dimensional shape may be used insofar as it has six substantially rectangular faces. The vertex angle of each "substantially rectangular" face has only to be approximately a right angle, and may deviate from a right angle preferably by approximately ±10 degrees, more preferably by approximately ±5 degrees. Such a packaging bag 10, being substantially a cuboid in shape, has an upper top face part 11, a lower bottom face part 12, and enclosing face parts 15 between the top face part 11 and the bottom face part 12. The enclosing face parts 15 has a front face part 15F in front, a back face part 15B in rear, and left and right face parts 15S. The front face part 15F is the face that faces the purchaser when the packaging bag 10 is displayed in a store in an ordinary manner, and, among the faces of the packaging bag 10, the front face part 15F shows information about the product (later described in detail) in the most noticeable manner.

The size of packaging bag 10 is approximately 80 mm to 300 mm in the left-right direction, 60 mm to 200 mm in the front-back direction, and 140 mm to 300 mm in the up-down direction, when two absorbent article arrays 1a and 1b, the upper array and the lower array, are contained therein.

The packaging bag 10 is made of a sheet material. The sheet material is not limited to a particular type, and resin, non-woven fabric, paper, and the like may be used. When the sheet material is resin, the sheet material may be a resin film such as a polyethylene film, a polypropylene film, and so forth. Also, the packaging bag 10 may be a single sheet in which two or more layers of the same sheet material or different sheet materials are joined together, such as a laminated film of paper and a resin film. Furthermore, the packaging bag 10 may be formed by stacking two or more layers of the same sheet material or different sheet materials without joining them together.

The packaging bag 10 may be dyed, partially or entirely. By dying the packaging bag 10, it is possible to prevent or substantially prevent the content of the absorbent article package 100, that is, absorbent articles 1, from being immediately visible. To dye the packaging bag 10, the sheet material that constitutes the packaging bag 10 may be color-printed, or a coloring agent may be mixed in the ingredients of the sheet material during manufacture of the sheet material. In the event a sheet is formed by laminating two or more sheet materials, the outer sheet material may be transparent, and the inner sheet material may be a dyed sheet material.

The packaging bag 10 is preferably dyed such that the dyed packaging bag 10 is opaque or translucent so that the inside cannot be seen through the dyed packaging bag 10. The color is not limited to a particular type, but colors that are perceived as beige, brown, black, gray, etc. are preferable. Furthermore, colors that have a saturation of preferably 8 or less, more preferably 6 or less, according to the Munsell color system, may be used. These colors are not very noticeable in an ordinary indoor environment, making the packaging bag less likely to be recognized as being one for absorbent articles.

Note that the external color of the absorbent articles (the color of the wrapping sheet if the absorbent articles are wrapped individually, or the color of the absorbent articles if the absorbent articles are not wrapped individually,) is not particularly limited either. The external color of the absorbent articles may be, for example, a color perceived as brown, black, gray, beige, or the like. Furthermore, a color that has a saturation of preferably 8 or less, more preferably 6 or less, according to the Munsell color system, may be used. There are also no particular limitations on the combination of the external color of the absorbent articles and the color of the packaging bag (the color of the dyed part 35). Here, if the external color of the absorbent articles contained inside and the color of the packaging bag are selected in a combination such that the user can immediately notice the difference of their colors, that is, for example, if the colors are selected such that the difference ΔE between the two colors is 4 or more, the external color of the absorbent articles becomes more noticeable through an inside check part 30, so that the user can find the inside check part 30. Therefore, it becomes easier to find a tearable part 20 that at least partially overlaps the inside check part 30, and the effect of reducing the effort and time required to open the packaging bag can be improved. Note that the above color difference ΔE is the color difference ΔE in the CIE1976 L*a*b* color space, and refers to ΔE=[(ΔL*)²+(Δa*)²+(Δb*)²]^{1/2}.

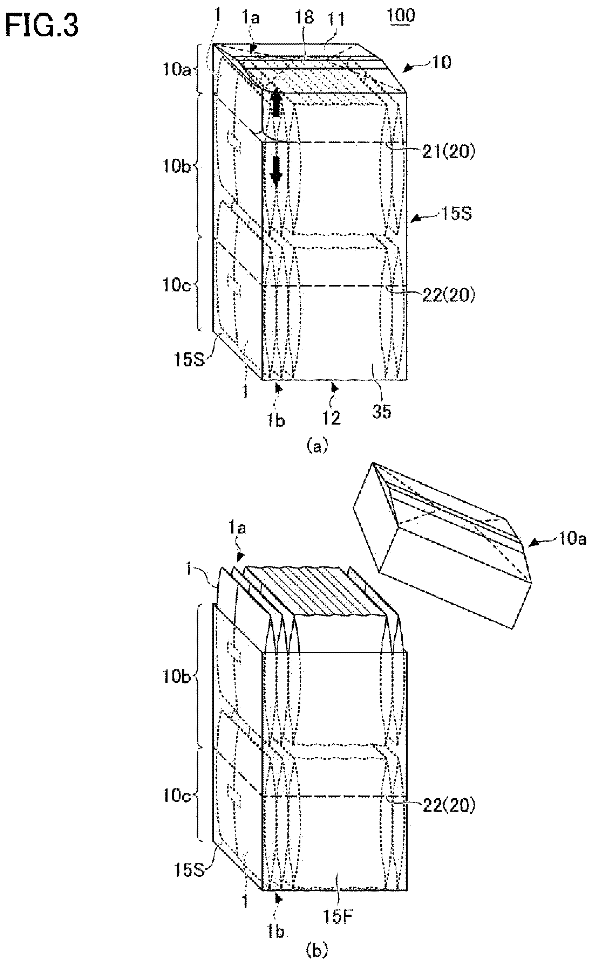

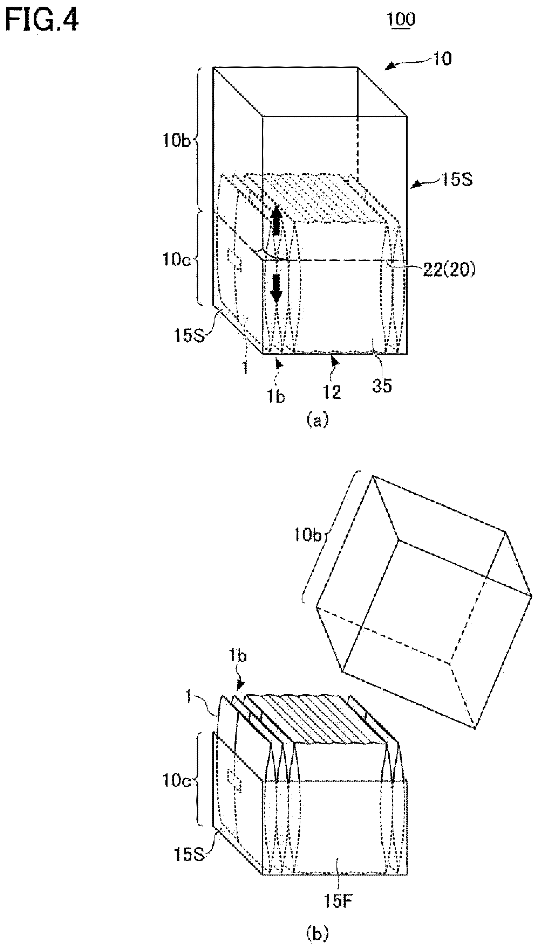

Also, changing the point of view from the one described above, the color of the packaging bag and the external color of the absorbent articles contained therein may be selected in such a combination that the user cannot immediately recognize the difference of their colors. For example, the color of the packaging bag and the external color of the absorbent articles may be selected such that the color difference ΔE between the two colors is less than 4. In that case, for example, assuming that the packaging bag is torn open and the upper part, or the upper part and the middle part, of the packaging bag are removed, and an upper portion of the absorbent articles is exposed (as illustrated in FIG. 3(b) or FIG. 4(b)), it is thus possible, for example, to make it difficult to distinguish between the packaging bag and the absorbent articles when viewed from the side, and make the external color of the absorbent articles inconspicuous in comparison to the color of the packaging bag. Therefore, for example, even if the absorbent article package is placed in a living room, its presence does not draw much attention.

The method packing for the packaging bag 10 is not particularly limited, but gusset packaging such as that shown in FIG. 1 is preferable. For example, the packaging bag 10 can be made by, for example, joining both edges of a long sheet material together and thus forming a long cylindrical sheet material, folding in both ends of the long cylindrical sheet material in the width direction, which is orthogonal to its longitudinal direction, and thus forming gussets, and joining together the portions of the sheet material folded in the width direction such that the portions of the sheet material adhere to each other in the direction of height, at a predetermined position in the longitudinal direction (at a position between absorbent article packages). Heat sealing, ultrasonic sealing, adhesive, etc. can be used for the joining of the sheet material. Therefore, although joint parts where the sheet material is joined are formed on the top face part 11 and the bottom face part 12 of the packaging bag 10, FIG. 1 shows only the joint part 18 formed in the top face part 11, and illustration of the joint part in the bottom face part 12 is omitted. Also, the structure of gazettes in the bottom face part 12 is also omitted from illustration. FIG. 2 shows an intended joint part 18a, which will later become the joint part 18. Intended joint parts 18a are also formed in the top face part 11 and the bottom face part 12, and formed between one side face part 15S and the back face part 15B.

### (Tearable parts)

As shown in FIG. 1 and FIG. 2, according to the present embodiment, tearable parts 20, which allow the packaging bag 10 to be torn in the up-down direction, are formed in the packaging bag 10. Saying that the packaging bag 10 can be torn in the up-down direction means that the parts, which the packaging bag 10 is divided into when torn from the tearable parts 20, are spaced apart in the up-down direction. In the examples shown in FIG. 1 and FIG. 2, the tearable parts 20 extend in directions that are orthogonal to the up-down direction, but the tearable parts 20 do not necessarily have to extend in directions that are orthogonal to the up-down direction (which will be described later in detail).

Each tearable part 20 is a seam-like part where the packaging bag 10 is made weak. For example, each tearable part 20 may be a perforation as shown in FIG. 1 and FIG. 2. The perforation refers to a seam-like part, in which cut parts, which are cuts in the sheet material that constitutes the packaging bag 10, and tie parts without cuts, are formed continuously and alternately. Note that the tearable parts 20 are not limited to perforations, and parts of the packaging bag 10 where the tearable parts 20 are formed may be made weak by reducing the thickness of the sheet material, changing the rigidity of the sheet material, or modifying the properties of the sheet material.

In the example shown in FIG. 1 and FIG. 2, multiple tearable parts 20 are provided vertically apart from each other. Among these multiple tearable parts 20, at least one tearable part may have different breaking strength from the rest of the tearable parts. For example, assume that the tearable parts 20 include a first tearable part 21 and a second tearable part 22, which are formed apart from each other in the up-down direction, and that the breaking strength of the first tearable part 21 and the breaking strength of the second tearable part 22 are different from each other.

For example, when the tearable parts 20 are perforations, the breaking strength of the tearable parts 20 can be reduced by increasing the length of each cut part, increasing the ratio of the cut parts to the tie parts, shortening the pitch of the cut parts, increasing the length of each tie part, reducing the ratio of the length of each tie part to the length of each cut part, increasing the ratio of the length of each cut part to the length of each tie part, and increasing the width of each cut part. As to how to reduce the breaking strength, if the tearable parts 20 are seam-like parts where the thickness is smaller, the thickness of these parts can be reduced accordingly. If the tearable parts 20 are formed by making corresponding parts of the packaging bag 10 weak by applying a chemical or the like, the range to apply the chemical to, or the amount of the chemical to apply, may be increased.

In the first embodiment shown in FIG. 1 and FIG. 2, the first tearable part 21 with relatively low breaking strength is formed in an upper part, and the second tearable part 22 with relatively high breaking strength is formed in a lower part. The first tearable part is formed within an extent of the upper absorbent article array 1a in the up-down direction, and the second tearable part 22 is formed within the extent of the lower absorbent article array 1b in the up-down direction.

In the examples of FIG. 1 and FIG. 2, the first tearable part 21 and the second tearable part 22 are both formed in an annular shape, and surround the packaging bag 10 circumferentially such that the up-down direction is the axis. That is, the first tearable part 21 and the second tearable part 22 are formed continuously, spanning the enclosing face parts 15, or, to be more specific, spanning the front face part 15F, the back face part 15B, and the two side face parts 15S. As a result of this, when the packaging bag 10 is torn along the entirety of one tearable part 20, the packaging bag 10 can be separated into the part above the tearable part 20 and the part below the tearable part 20.

How the packaging bag 10 is torn open at the tearable parts 20 will be further described with reference to FIG. 3 and FIG. 4. FIG. 3 shows a case in which the packaging bag 10 is torn open at the first tearable part 21, and FIG. 4 shows a case in which the packaging bag 10 is torn open at the second tearable part 22.

The edge between two enclosing face parts 15 (the boundary between two enclosing face parts 15) or parts around the edge are easy to grip when opening the absorbent article package, and so many users tear open the packaging bag 10 by gripping two neighboring enclosing face parts 15. For example, referring to FIG. 3(a), around the edge between the front face part 15F and one side face part 15S (the boundary between the front face part 15F and one side face part 15S), the user can hold a part (upper part 10a) above the first tearable part 21 with one hand and hold a part (middle part 10b) below the first tearable part 21 with the other hand, and pull these parts upward and downward, as indicated by the arrows. By this means, it is possible to tear open the sheet material of the packaging bag 10 at the tearable part 21, so that the packaging bag 10 can be separated into the upper part 10a, the middle part 10b, and a lower part 10c (see FIG. 3(b)). The upper part 10a may be removed, or may be kept above the lower part 10c after the separation and removed every time an absorbent article 1 is taken out. This allows the upper part 10a to function as a cover. After the upper part 10a is removed, the upper portion of the array 1a of the absorbent articles 1 of the upper level is exposed from the remaining middle part 10b, so that the absorbent articles 1 can be taken out with ease. For example, since the user can pick up an absorbent article 1 without touching the packaging bag 10, the noise generated when the packaging bag 10 is moved is also reduced.

FIG. 4(a) shows a state after all of the absorbent articles 1 of the upper absorbent article array 1a have been taken out. The user may take out absorbent articles 1 from the lower absorbent article array 1b in this state, but the user has to put his/her hand in the middle part 10b of the packaging bag 10. However, the packaging bag 10 can be torn open at the second tearable part 22 as well. Similar to or the same as the case of tearing open the packaging bag 10 from the first tearable part 21, the second tearable part 22 can be torn by gripping parts near the boundary between two neighboring enclosing face parts 15 and pulling these parts upward and downward, as indicated by the arrows. Once the tearing along the second tearable part 22 is completed, the middle part 10b can be removed (see FIG. 4(b)). The upper portion of the array 1b of the absorbent articles 1 of the lower level is exposed from the lower part 10c, which is left after the middle part 10b is removed. This makes it easy to take out the absorbent articles 1 of the lower level with ease.

Assuming that the absorbent article package 100 contains multiple absorbent articles in upper and lower arrays, in many cases, as shown in FIG. 3 and FIG. 4, the packaging bag 10 is torn open at the upper tearable part so that the upper absorbent article array 1a is used first (see FIG. 3(a)). However, even if the user intends to tear the upper tearable part alone, depending on how the user uses his/her force, or how much force he/she uses, to tear the upper tearable part, the force might transmit to the lower tearable part, and the lower tearable part, that is, an unintended part, might be torn. If the packaging bag is torn open at an unintended tearable part (that is, in the examples illustrated, if the second tearable part is torn), the packaging bag has a hole in it, and its functionality as a container is impaired, which is a deterioration. By contrast with this, according to the present embodiment, the first tearable part 21 with relatively low breaking strength is formed in the upper part, and the second tearable part 22 with relatively high breaking strength is formed in the lower part, so that the user can tear the upper tearable part (that is, tear only the first tearable part 21) without being too careful not to tear the lower tearable part. Furthermore, it is possible to prevent or substantially prevent the part below the first tearable part 21, that is, the middle part 10b and the lower part 10c, from being torn, so that the middle part 10b and the lower part 10c, which remain after the upper part 10a is separated, can maintain their function as a container.

Each of the tearable parts 20 (at least the first tearable part 21 and the second tearable part 22) is formed within an extent of one absorbent article array in the up-down direction, and it is still preferable to form each tearable part 20 above the center of an absorbent article array in the up-down direction. For example, referring to the examples illustrated in FIG. 1 to FIG. 4, the first tearable part 21 in the upper part may preferably be formed at a position above the center of the absorbent article array 1a in the up-down direction, and the second tearable part 22 in the lower part may preferably be formed at a position above the center of the absorbent article array 1b in the up-down direction. Assuming that the length or extent of an absorbent article array in the up-down direction is 100%, a tearable part 20 may be formed preferably at a position corresponding to 60% to 90%, more preferably at a position corresponding to 70% to 80%, even more preferably at a position corresponding approximately to 75%, from the bottom position of the absorbent article array. If a tearable part 20 is formed at such a position relative to an absorbent article array or absorbent articles, when the packaging bag 10 is torn open from the tearable part 20 in the up-down direction, parts for gripping the packaging bag 10 can be secured above and below the tearable part. Also, it is possible to secure a lower part having a certain length in the up-down direction, which is the remaining part of the packaging bag 10 after the tearable part 20 is torn, thereby preventing or substantially preventing a situation where the absorbent articles 1 are exposed too much, the remaining part of the packaging bag 10 is unable to support the absorbent articles 1 or the absorbent article array, and the absorbent articles 1 spill out.

In the examples shown in FIG. 1 to FIG. 4, the tearable parts 20 (the first tearable part 21 and the second tearable part 22) are formed such that both tearable parts span the entirety of the enclosing face parts 15 in a continuous manner, but the tearable parts 20 may be formed to cover only part of the faces of the enclosing face parts 15. For example, the tearable parts 20 may be formed to span three faces, namely the front face part 15F and the side face parts 15S, and not formed in the back face part 15B. For example, if the upper first tearable part 21 is formed to span three faces of the front face part 15F and the side face parts 15S and not formed in the back face part 15B, the upper part 10a in FIG. 3(a) is not completely separated, and may remain connected with the middle part 10b in the back face part 15B. In that case, a horizontal fold line is formed in the back face part 15B, at the height of the terminal end of the upper first tearable part 21 (at a position corresponding to the first tearable part 21 in the up-down direction, at the boundary between the side face part 15S and the back face part 15B), allowing the upper part 10a to pivot about the fold line. By tilting the upper part 10a to the rear, the absorbent article array 1a can be exposed. By tilting the upper part 10a forward, the absorbent article array 1a can be covered.

A tearable part 20 does not necessarily have to be continuous, and may, for example, include discontinuous parts in one face and be formed as multiple horizontal segments. In that case, the packaging bag 10 may be used without tearing the discontinuous parts, or the discontinuous parts may be torn after the weakened parts are torn. Also, multiple tearable parts 20 may be formed close to each other in the up-down direction. In this case, multiple starting points from which the packaging bag 10 can be torn in the up-down direction are formed in the up-down direction, so that it becomes even easier to tear open the packaging bag 10.

### <Second embodiment>

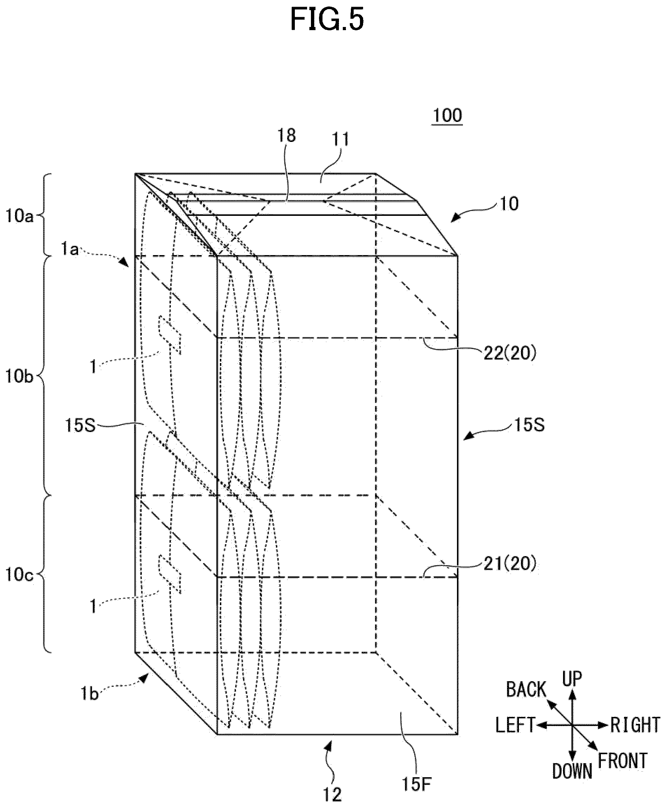

In the second embodiment, as shown in FIG. 5, a first tearable part 21 with relatively low breaking strength is formed in a lower part, and a second tearable part 22 with relatively high breaking strength is formed in an upper part. Compared to the first embodiment, the breaking strength of two upper and lower tearable parts 20 is reversed. Therefore, it is more difficult to tear open the packaging bag 10 from the upper part than tear open the packaging bag 10 from the lower part. The present embodiment is suitable when the user wants to open the absorbent article package 100 from the lower part. For example, some users might take out all of the absorbent articles 1 contained in the packaging bag 10 and put them in another container specifically designed for that purpose. Examples of such users include a user who has an absorbent article container of his/her favorite design, a user who has a special container that is designed for installation in a store's toilet, and so forth. In such cases, the packaging bag 10 may be torn open at the tearable part formed near the lower end, and all of the absorbent articles 1 contained inside may be dropped and removed at once from the bottom face part 12 of the packaging bag 10. To do so the user might want to tear a tearable part formed in a lower part of the packaging bag 10 first, that is, a tearable part formed near the bottom face part 12 of the packaging bag 10. If the first tearable part 22 with relatively low breaking strength serves as the lower tearable part, the second tearable part 22 with relatively high breaking strength is then naturally the upper one, so that the user can tear only the lower first tearable part 21 of the packaging bag 10, without being too careful not to tear the upper tearable part. By this means, the packaging bag 10 can be opened quickly from the bottom face part 12.

Also, the present embodiment provides advantages when manufacturing the absorbent article package 100. In manufacture of the absorbent article package 100, a long wrapping sheet material, which will later become the packaging bag 10, is formed into a cylindrical shape, and this long, cylindrical sheet material is cut to a predetermined length in a direction that is orthogonal to the direction in which the sheet material is transported. Then, one side of the cut wrapping sheet material, namely the top face part 11 of the packaging bag 10 is joined, and the absorbent articles 1 are packed in the direction from the bottom face part 12 to the top face part 11 of the packaging bag 10. The impact that is produced by this packing process tends to transmit to the top face part 11 more strongly, and so there is a possibility that the tearable part 20 formed near the top face part 11 (the upper part) is torn unintentionally. Nevertheless, if the tearable part 20 near the top face part 11 is the second tearable part 22 with relatively high breaking strength, it is possible to reduce the possibility that the packaging bag 10 will be torn by the impact of the packing of the absorbent articles 1.

### <Variations>

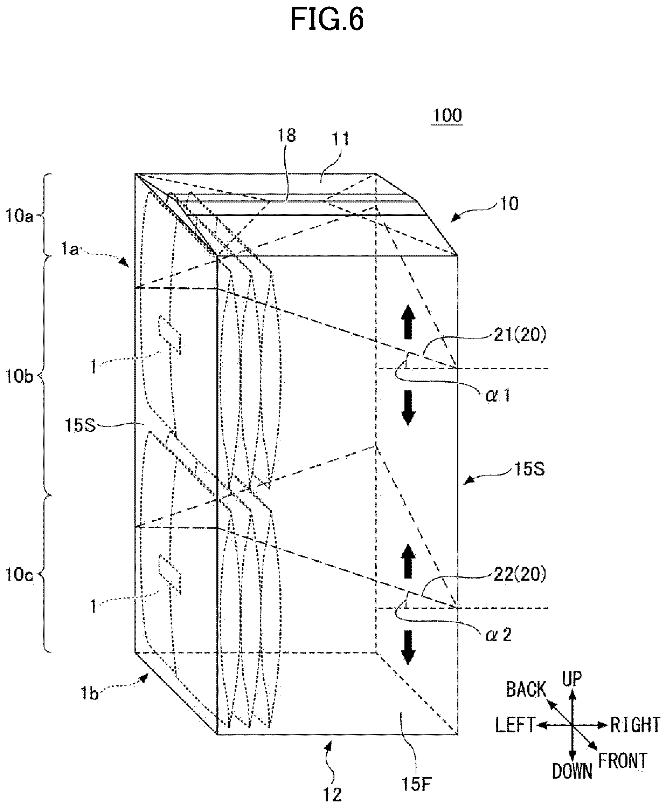

Variations of the absorbent article package 100 will be described below. For example, FIG. 6 shows a variation of the absorbent article package 100 of the first embodiment (FIG. 1). In this example, again, the first tearable part 21 with relatively low breaking strength is formed in an upper part, and the second tearable part 22 with relatively high breaking strength is formed in a lower part.

In the example shown in FIG. 6, the tearable part 20 (the first tearable part 21 and the second tearable part 22) does not extend in a direction (the left-right direction or the front-back direction) that is orthogonal to the up-down direction as in the examples shown in FIG. 1 and FIG. 2. In this example, the tearable parts 20 (the first tearable part 21 and the second tearable part 22) are formed at an angle with respect to the above orthogonal direction. To be more specific, each tearable part 20 can be formed, for example, to span two neighboring enclosing face parts 15, and to rise upward or fall downward as it gets nearer to the boundary between the two enclosing face parts 15.

In the example shown in FIG. 6, the first tearable part 21, which is the upper tearable part, is formed to span the front face part 15F and the left side face part 15S, and rise upward as it gets nearer to the boundary between the front face part 15F and left side face part 15S. In other words, the upper first tearable part 21 has an upwardly oriented vertex at the boundary between the front face part 15F and the left side face part 15S. Also, the first tearable part 21 is also formed to span the front face part 15F and the right side face part 15S, and fall downward as it gets nearer to the boundary between the front face part 15F and the right side face part 15S. That is, the upper first tearable part 21 has a downwardly oriented vertex at the boundary between the front face part 15F and the right side face part 15S. Furthermore, in the example illustrated, the first tearable part 21 is formed to fall downward as it gets nearer to the boundary between the back face part 15B and the left side face part 15S, and rise upward as it gets nearer to the boundary between the back face part 15B and the right side face part 15S. However, the first tearable part 21 may be formed in a direction that is orthogonal to the up-down direction, in the back face part 15B and both side face parts 15S.

Furthermore, the second tearable part 22, which is the lower tearable part, is also formed to span the front face part 15F and the left side face part 15S, like the upper first tearable part 21, and rise upward as it gets nearer to the boundary between the front face part 15F and the left side face part 15S. Furthermore, the second tearable part 22 is formed to fall downward as it gets nearer to the boundary between the front face part 15F and the right side face part 15S, fall downward as it gets nearer to the boundary between the back face part 15B and the left side face part 15S, and rise upward as it gets nearer to the boundary between the back face part 15B and the right side face part 15S.

In this way, in the event each tearable part 20 extends to span two neighboring enclosing face parts 15 and rises upward or falls downward as it gets nearer to the boundary between the two enclosing face parts 15, if the user pulls the packaging bag 10 upward and downward by gripping parts around the tearable part 20, the stress tends to concentrate on a vertex located at a boundary between the two neighboring enclosing face parts 15, so that the user can tear open the packaging bag 10 from the boundary between the two enclosing face parts 15, with relatively little force. Also, if a tearable part 20 protrudes upward or downward at the boundary between two enclosing face parts 15, the user can find the tearable part 20 with ease. This also improves the ease of tearing.

Furthermore, in the event a tearable part 20 is formed obliquely to a direction that is orthogonal to the up-down direction (thus forming an upward or downward angle), if the tearable part 20 is torn and then the upper part that is torn apart is removed, a part of the remaining part of the packaging bag 10 can be heightened (can be made longer in the up-down direction). For example, after the upper first tearable part 21 is torn, a part of the middle part 10b, which remains after the upper part 10a is removed, can be heightened. By this means, even if the absorbent articles 1 that are erect upright fall down, the absorbent articles 1 can still be supported, thereby preventing or substantially preventing the absorbent articles 1 from spilling out of the packaging bag.

Note that, when the user wants to tear open the packaging bag 10 at a tearable part 20, although he/she can do so from any part of the packaging bag 10, the user tends to use force with the hand that grips an upper part of the packaging bag 10, and therefore, in many cases, the user tears open the packaging bag 10 from a point in an upper part of the packaging bag 10 where the length in the up-down direction is long. For example, referring to the boundary between the front face part 15F and the right side face part 15S in the example of FIG. 6, the upper first tearable part 21 falls downward as it gets nearer to the boundary, and has a downwardly oriented vertex, and so the part above the first tearable part 21 is relatively long in the up-down direction. Therefore, the user can hold parts above and below the first tearable part 21 with both hands and pull them upward and downward as indicated by the arrows. Similarly, when the user tears open the packaging bag 10 from the second tearable part 22, the user can do so with ease by tearing open the packing bag 10 in the up-down direction at a location where a boundary between the front face part 15F and the right side face part 15S is formed.

Note that the angle that the tearable part 20 forms with respect to the direction that is orthogonal to the up-down direction may be preferably 10 degrees to 40 degrees, more preferably 20 degrees to 30 degrees. Also, the tearable part 20 may be tilted either upward or downward with respect to the orthogonal direction. In the example shown in FIG. 6, the angle α1 that the first tearable part 21 forms with respect to the orthogonal direction and the angle α2 that the second tearable part 22 forms with respect to the orthogonal direction may be both preferably 10 degrees to 40 degrees, more preferably 20 degrees to 30 degrees. The angle α1 and the angle α2 may be the same or different.

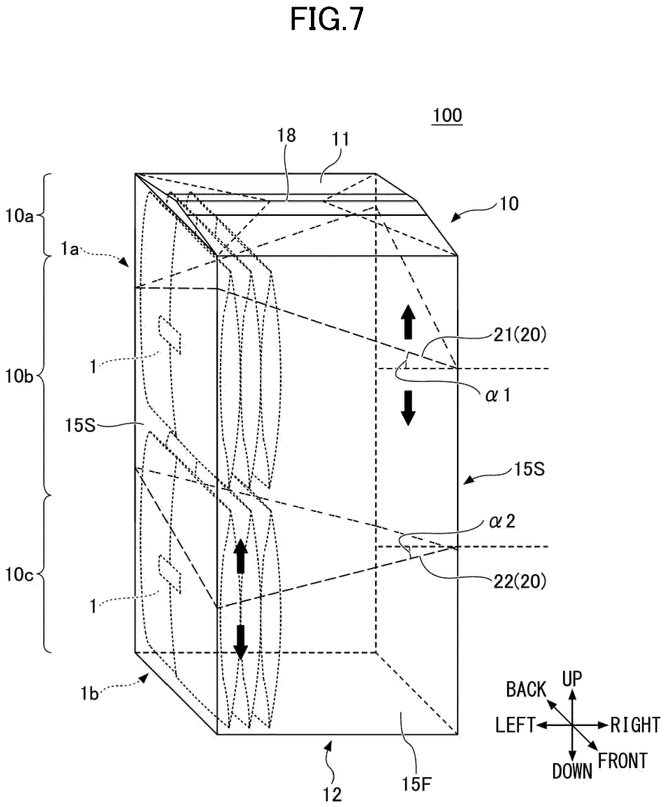

FIG. 7 shows another variation of the tearable parts 20. Like the example shown in FIG. 6, the example shown in FIG. 7 is also a variation of the first embodiment (FIG. 1), in which the first tearable part 21 with relatively low breaking strength is formed in an upper part, and the second tearable part 22 with relatively high breaking strength is formed in a lower part. The first tearable part 21 of FIG. 7 is formed the same or substantially the same as the first tearable part 21 shown in FIG. 6. On the other hand, the second tearable part 22 falls downward as it gets nearer to the boundary between the front face part 15F and the left side face part 15S. Also, the second tearable part 22 is formed to rise upward as it gets nearer to the boundary between the front face part 15F and the right side face part 15S, rise upward as it gets nearer to the boundary between the back face part 15B and the left side face part 15S, and fall downward as it gets nearer to the boundary between the back face part 15B and the right side face part 15S.

The working of the second tearable part 22 in the example shown in FIG. 7 is the same or substantially the same as that of the second tearable part 22 shown in FIG. 6. However, when the user tears open the packaging bag 10 from the second tearable part 22, the user can tear open the packaging bag 10 with ease by, for example, pulling parts of the packaging bag 10 near the boundary between the front face part 15F and the left side face part 15S, upward and downward, as indicated by the arrows.

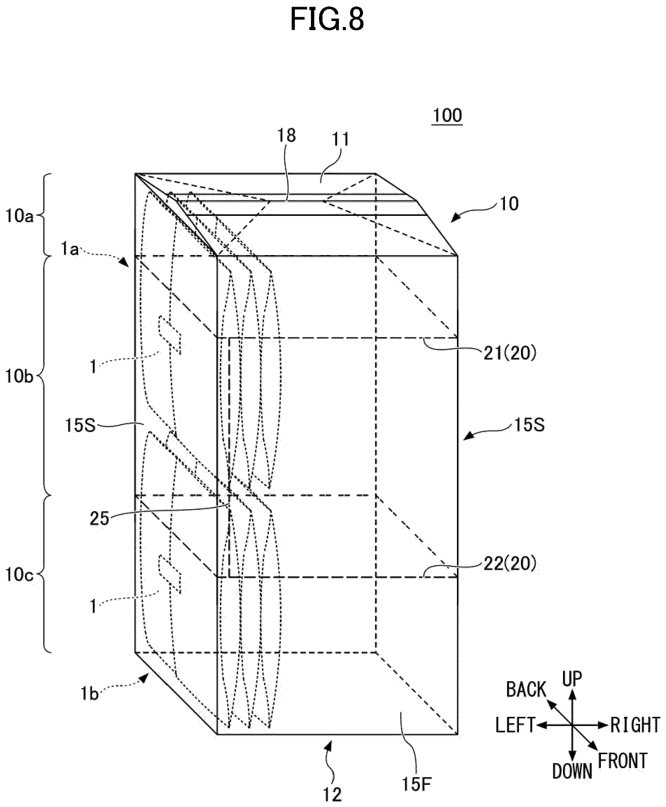

FIG. 8 shows yet another variation. In the example shown in FIG. 8, in addition to the tearable parts 20 (the first tearable part 21 and the second tearable part 22) shown in FIG. 1, a spare tearable part 25 is formed between the first tearable part 21 and the second tearable part 22. The spare tearable part 25 is formed to extend in the up-down direction. It is preferable if the spare tearable part 25 is formed using the same or substantially the same method as that of the tearable parts 20, and perforated like the tearable parts 20. Also, it is preferable if the spare tearable part 25 is connected with both of the first tearable part 21 and the second tearable part 22.

The spare tearable part 25 helps tearing the second tearable part 22 when, for example, the packaging bag 10 is torn at the first tearable part 21 and then the upper part 10a is removed (see FIG. 3(b)). That is, before the second tearable part 22 is torn, the middle part 10b may be torn in advance along the spare tearable part 25, that is, in the up-down direction, so that the user can grip and pull either one of the torn edges of the middle part 10b and tear the second tearable part 22 further. If the spare tearable part 25 is not torn, the tear at the second tearable part 22 develops in two directions, or the tear develops at two tip positions (see FIG. 4(a)). On the other hand, if the middle part 10b is separated by the spare tearable part 25 in the up-down direction, the second tearable part 22 can be torn in one direction, making the tearing even easier.

In the example shown in FIG. 8, the spare tearable part 25 is formed near the boundary between the front face part 15 and the left side face part 15S, but the position where the spare tearable part 25 is formed is not particularly limited. For example, although the spare tearable part 25 is located near the boundary between the front face part 15 and the left side face part 15S, it may be formed in the left side face part 15S, not in the front face part 15, or it may be formed near the boundary between the front face part 15 and the right side face part 15S. Also, the spare tearable part 25 may be formed near the center of one enclosing face part 15 in the left-right direction or the front-back direction. For example, the spare tearable part 25 may be formed near the center of the front face part 15 in the left-right direction. In order not to impair the visibility of the information about the product affixed to the packaging bag 10, it is preferable to form the spare tearable part 25 in an inconspicuous position.

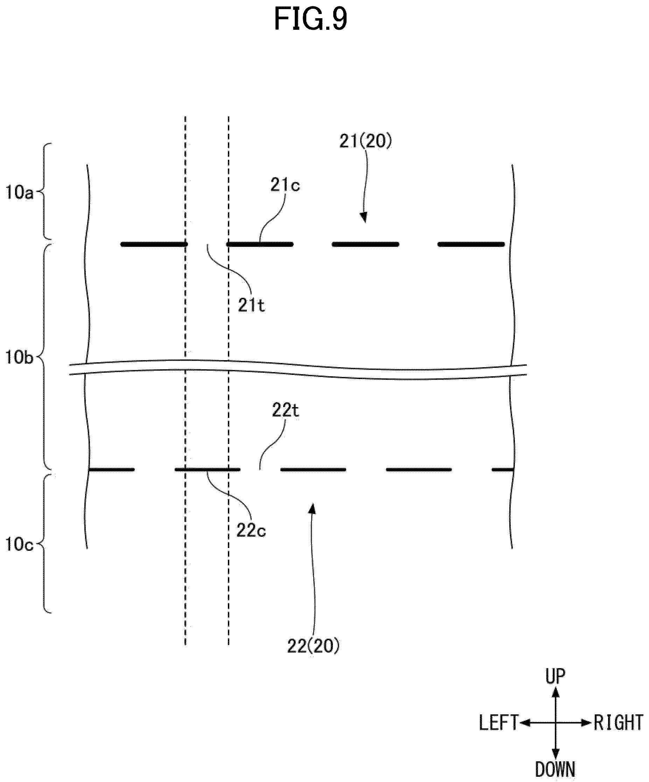

FIG. 9 shows a still another variation of the tearable parts 20. FIG. 9 is a partially enlarged view corresponding to part I in FIG. 1. In FIG. 9, part around the center of the middle part 10b in the up-down direction is omitted. In the example shown in FIG. 9, the first tearable part 21 and the second tearable part 22 are both perforations in which cut parts and tie parts are repeated alternately. In this example, for ease of explanation, the length and pitch of each cut part 21c of the first tearable part 21 are the same as the length and pitch of each cut part 22c of the second tearable part 22. That is, the length and pitch of the tie parts 21t of the first tearable part 21 are the same as the length and pitch of the tie parts 22t of the second tearable part 22. Note that, in this example, the width of the cut parts 21c of the first tearable part 21 is wider than the width of the cut parts 22c of the second tearable part 22. As a result of this, the breaking strength of the first tearable part 21 is lower than the breaking strength of the second tearable part 22. This structure can be obtained, for example, by cutting out the cut parts 21c, when forming the cut parts 21c in the first tearable part 21, such that the cut parts 21 have a certain width. However, referring to the example of FIG. 9, the perforation of the first tearable part 21 and the perforation of the second tearable part 22 are out of phase. In FIG. 9, when two virtual lines (the dotted lines in FIG. 9) are drawn in the up-down direction at both ends of a tie part 21t in the first tearable part 21, the two virtual lines overlap a cut part 21c in the second tearable part 22.

As described above, during manufacture of the absorbent article package 100, normally, the absorbent articles 1 are packed in the packaging bag 10 from the bottom face part 12. Now, if the absorbent articles 1 are individually-wrapped articles wrapped with a wrapping sheet and each individually-wrapped article's wrapping sheet has sharp edge parts (edges), the individually-wrapped articles may get caught in the cut parts of the perforations in the above process of packing the absorbent articles 1. In particular, individually-wrapped articles to be placed in the upper level of the packaging bag 10 must move inside the packaging bag 10 and pass the second tearable part 22 and the first tearable part 21 in order. In that case, if the cut parts 21c of the first tearable part 21 and the cut parts 22c of the second tearable part 22 are formed in the same positions in a direction that is orthogonal to the up-down direction (for example, the left-right direction), one individually-wrapped article's edge parts might get caught in both the cut parts 21c of the first tearable part 21 and the cut parts 22c of the second tearable part 22, and therefore the possibility that the packaging bag 10 is torn during manufacture, more specifically during the process of packing individually-wrapped articles, increases. By contrast with this, as shown in FIG. 9, by positioning the perforation of the first tearable part 21 and the perforation of the second tearable part 22 to be out of phase, and making the cut parts 21c of the first tearable part 21 and the cut parts 22c of the second tearable part 22 not aligned in the up-down direction, it is possible to reduce the possibility that an individually-wrapped article's edge parts get caught in both the cut parts 21c of the first tearable part 21 and the cut parts 22c of the second tearable part 22.

Although the present invention has been described above based on embodiments, the present invention is by no means limited to these embodiments. Various changes, alterations, substitutions, additions, omissions, combinations, and so forth may be applicable to the above embodiment within the scope of the claims, and all such changes still belong to the technical scope of the present invention. Also, the components described hereinabove can be combined flexibly.

Specific examples of the present invention will be described below.

### (Note 1)

An example according to note 1 is an absorbent article packaging bag configured to contain two or more absorbent article arrays in an up-down direction, each one of the two or more absorbent article arrays being formed with a plurality of absorbent articles that stand erect and are aligned, and, in this absorbent article packaging bag:
the absorbent article packaging bag is substantially a cuboid in shape, with a top face part, a bottom face part, and enclosing face parts connecting the top face part and the bottom face part,
tearable parts, from which the enclosing face parts can be torn in the up-down direction, are formed in the enclosing face parts,
the tearable parts include:
   a first tearable part, formed within an extent of one of the two or more absorbent article arrays in the up-down direction; and
   a second tearable part, formed apart from the first tearable part in the up-down direction, and formed within an extent of an absorbent article array other than the one of the two or more absorbent article arrays in the up-down direction, and
the first tearable part has lower breaking strength than the second tearable part.

The example of note 1 is a packaging bag that can contain two or more upper and lower absorbent article arrays and that therefore can contain a large number of absorbent articles. Furthermore, in the packaging bag according to the present embodiment, a first tearable part, formed within an extent of one of the two or more absorbent article arrays in the up-down direction, and a second tearable part, formed within an extent of an absorbent article array other than the one of the two or more absorbent article arrays are formed in the enclosing face parts. Consequently, by tearing open the packaging bag at the tearable part located uppermost among the tearable parts formed in the packaging bag, for example, it is possible to expose the absorbent article array corresponding to the position where the uppermost tearable part is formed, and take out the absorbent articles included in that absorbent article array with ease. After the absorbent articles of the exposed absorbent article array have been used, the packaging bag is torn open at the tearable part that is located below the uppermost tearable part, so that it is possible to expose the absorbent article array corresponding to the position where the lower tearable part is formed, and take out the absorbent articles included in that absorbent article array with ease.

Furthermore, in this example, the breaking strength varies between the first tearable part and the second tearable part, which are spaced apart in the up-down direction. Because of this, in the packaging bag, the first tearable part is more easily torn than the second tearable part, and the other tearable part is more difficult to be torn. Therefore, for example, it is possible to achieve advantages when tearing open the packaging bag at the beginning of the use of the packaging bag or an absorbent article package in which absorbent articles are contained in the packaging bag (when opening the packaging bag, etc.). In other words, among the first tearable part and the second tearable part, the packaging bag may be opened from the first tearable part where the breaking strength is weaker, so that it is possible to tear open the packaging bag with ease, and avoid or substantially avoid tearing the second tearable part where the breaking strength is greater.

### (Note 2)

In an example according to note 2, the first tearable part is formed above the second tearable part.

According to the example of note 2, the first tearable part with relatively low breaking strength is formed above the second tearable part with relatively high breaking strength, so that it is possible to tear open the packaging bag at an upper part (near the top face part). Most users who use the absorbent article package in which two or more upper and lower absorbent article arrays are contained use the upper-level absorbent articles first. Therefore, when starting to use the absorbent article package, the user tears open the packaging bag from the upper first tearable part formed in the packaging bag. Assuming that the breaking strength is the same between the first tearable part and the second tearable part, if the user intends to tear the first tearable part alone, depending on how the user uses his/her force, or how much force he/she uses, to tear the first tearable part, the force might transmit to the lower second tearable part, and the second tearable part, that is, an unintended part, might be torn. If the packaging bag is torn open at the second tearable part, which is an unintended part, the part of the packaging bag that remains after the part above the first tearable part is removed is torn, that is, the remaining part of packaging bag has a hole, and its functionality as a container is impaired, which is a deterioration. By contrast with this, according to this example, the upper first tearable part is easier to tear, so that the user can tear only the upper first tearable part, without being too careful not to tear the lower second tearable part. Therefore, the entire part below the first tearable part can function as a container without a torn part.

### (Note 3)

In an example according to note 3, the first tearable part is formed below the second tearable part.

According to the example of note 3, the first tearable part with relatively low breaking strength is formed below the second tearable part with relatively high breaking strength, so that it is easier to tear open the packaging bag at a lower part (near the bottom face part). For example, some users might take out all of the absorbent articles contained in the packaging bag and put them in another container specifically designed for that purpose. In these cases, the users might want to tear open the packaging bag from the lower tearable part, and drop and remove all of the absorbent articles contained inside at once from the bottom of the packaging bag 10. According to this example, the first tearable part with lower breaking strength is located in a lower part, so that the user can tear only the lower first tearable part, without being too careful not to tear the second tearable part located in an upper part. This allows the user to open the packaging bag from the lower part (from near the bottom face part) quickly.

### (Note 4)

In an example according to note 4, at least one tearable part from among the tearable parts is formed to span two neighboring enclosing face parts and rises upward or falls downward as the one tearable part gets nearer to a boundary between the two enclosing face parts.

When the user tears open the packaging bag at a tearable part, the user tears open the packaging bag from parts of the packaging bag that are easy to hold, and these parts are often found near edges of the packaging bag, which is substantially a cuboid in shape, that is, near boundaries between two enclosing face parts. By contrast with this, according to the example of note 4, a tearable part is formed to span the boundary between two enclosing face parts, and rise upward as it gets nearer to the boundary or fall downward as it gets nearer to the boundary. Therefore, the two tearable parts have upwardly or downwardly oriented vertices at boundaries between enclosing face parts. When the user pulls the packaging bag upward and downward by gripping parts above and below a tearable part, the stress tends to concentrate on the corresponding vertex of the tearable part, so that the force needed for the tearing can be reduced. Also, since the tearable parts' vertices formed near the boundaries between two enclosing face parts are easy to find, the user can find the tearable parts with ease.

### (Note 5)

In an example according to note 5, a spare tearable part that runs in the up-down direction is provided between the first tearable part and the second tearable part.

The spare tearable part according to note 5 is formed in the up-down direction to connect the first tearable part and the second tearable part, so that the part (middle part) of the packaging bag between the first tearable part and the second tearable part can be torn in the up-down direction of the packaging bag. It then follows that, assuming an example in which the first tearable part is formed above the second tearable part, when the users opens the packaging bag by tearing the first tearable part and, after the absorbent articles in the upper absorbent article array have been used, tries to tear the second tearable part, the user can tear the middle part (part in the packaging bag between the first tearable part and the second tearable part) along the spare tearable part before tearing the second tearable part. If the packaging bag is torn at the spare tearable part in advance, the packaging bag then can be torn along the second tearable part by pulling either end of the middle part after the spare tearable part is torn, so that the packaging bag can be torn in one direction from the point where the tear starts. This makes it easy to remove the middle part.

### (Note 6)

In an example according to note 6, the tearable parts include perforations, and the perforation of the first tearable part and the perforation of the second tearable part are positioned so as to be out of phase.

The perforations are structured such that the cut parts, where the sheet material forming the packaging bag is cut, and the tie parts, where the sheet material is not cut, continue alternately. Assuming that individually-wrapped articles are provided by wrapping absorbent articles with a wrapping sheet, when individually-wrapped articles are packed in a packaging bag during manufacture of an absorbent article package, the individually-wrapped articles placed in the upper part of the packaging bag must move inside the packaging bag by passing both positions of the first tearable part and the second tearable part. In this case, if the positions of cut parts in the first tearable part and the positions of cut parts in the second tearable part are the same in the left-right direction (horizontal direction), there is a possibility that one individually-wrapped article's edge parts get caught in both the cut parts of the first tearable part and the cut parts of the second tearable part, which then leads to an increased possibility that the packaging bag is torn during the process of packing individually-wrapped articles in the packaging bag during manufacture. By contrast with this, according to the example of note 6, the perforation of the first tearable part and the perforation of the second tearable part are positioned out of phase, that is, the cut parts of the first tearable part and the cut parts of the second tearable part are shifted in the left-right direction such that their positions do not overlap in the left-right direction, so that it is possible to reduce the possibility that one individually-wrapped article's edge parts get caught by both the cut parts of the first tearable part and the cut parts of the second tearable part.

### (Note 7)

The example of note 7 is an absorbent article package containing a plurality of absorbent articles in the absorbent article packaging bag of any one of note 1 to note 6.

According to the example of note 7 above, it is possible to provide an absorbent article package that provides the same advantages as any of the examples according to note 1 to note 6.

This application is based on and claims priority to Japanese Patent Application No. 2022-146677, filed on September 15, 2022, the entire contents of which are incorporated herein by reference.

### DESCRIPTION OF THE REFERENCE NUMERALS

- 1: absorbent article (incontinence pad)
- 1a, 1b: absorbent article array
- 10: absorbent article packaging bag (packaging bag)
- 10a: upper part
- 10b: middle part
- 10c: lower part
- 11: top face part
- 12: bottom face part
- 15: enclosing face part
- 15F: front face part
- 15B: back face part
- 15S: side face part
- 18: joint part
- 18a: intended joint part
- 20, 21, 22: tearable part
- 25: spare tearable part
- 100: absorbent article package

## Claims

1. An absorbent article packaging bag configured to contain two or more absorbent article arrays in an up-down direction, each one of the two or more absorbent article arrays being formed with a plurality of absorbent articles that stand erect and are aligned,
wherein the absorbent article packaging bag is substantially a cuboid in shape, with a top face part, a bottom face part, and enclosing face parts connecting the top face part and the bottom face part,
wherein tearable parts, from which the enclosing face parts can be torn in the up-down direction, are formed in the enclosing face parts,
wherein the tearable parts include:
a first tearable part, formed within an extent of one of the two or more absorbent article arrays in the up-down direction; and
a second tearable part, formed apart from the first tearable part in the up-down direction, and formed within an extent of an absorbent article array other than the one of the two or more absorbent article arrays in the up-down direction, and
wherein the first tearable part has lower breaking strength than the second tearable part.

2. The absorbent article packaging bag according to claim 1, wherein the first tearable part is formed above the second tearable part.

3. The absorbent article packaging bag according to claim 1, wherein the first tearable part is formed below the second tearable part.

4. The absorbent article packaging bag according to claim 1, wherein at least one tearable part from among the tearable parts is formed to span two neighboring enclosing face parts and rises upward or falls downward as the one tearable part gets nearer to a boundary between the two enclosing face parts.

5. The absorbent article packaging bag according to claim 1, wherein a spare tearable part that runs in the up-down direction is provided between the first tearable part and the second tearable part.

6. The absorbent article packaging bag according to claim 1,
wherein the tearable parts include perforations, and
wherein a perforation of the first tearable part and a perforation of the second tearable part are positioned so as to be out of phase.

7. An absorbent article package containing a plurality of absorbent articles in the absorbent article packaging bag of any one of claims 1 to 6.
